(19) Europäisches Patentamt
European Patent Office
Office européen des brevets

(11) Veröffentlichungsnummer: **0 457 140 A2**

# (12) EUROPÄISCHE PATENTANMELDUNG

(21) Anmeldenummer: **91107254.4**

(22) Anmeldetag: **04.05.91**

(51) Int. Cl.5: **C07C 311/29**, C07C 311/49, C07D 211/96, C07D 231/12, C07D 231/16, C07D 295/26, C07D 213/86, C07D 307/68, C07D 265/30, A01N 41/06, A01N 43/56

(30) Priorität: **17.05.90 DE 4015834**

(43) Veröffentlichungstag der Anmeldung: **21.11.91 Patentblatt 91/47**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Haug, Michael, Dr.**
**Fahner Weg 5**
**W-5060 Bergisch Gladbach 2(DE)**
Erfinder: **Haas, Wilhelm, Dr.**
**Nordstrasse 1**
**W-5014 Kerpen 3(DE)**
Erfinder: **Pfister, Theodor, Dr.**
**Lichtenbergerstrasse 30**
**W-4019 Monheim(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Schmidt, Robert R., Dr.**
**Im Waldwinkel 110**
**W-5060 Bergisch Gladbach 2(DE)**
Erfinder: **Krauskopf, Birgit, Dr.**
**Kicke 19**
**W-5060 Bergisch Gladbach 1(DE)**

## (54) Phenoxyphenylsulfonylverbindungen.

(57) Beschrieben werden neue Phenoxyphenylsulfonylverbindungen der Formel (I)

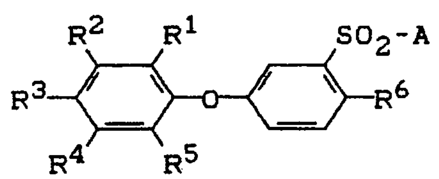

(I)

in welcher

$R^1$ bis $R^6$ und A die in der Beschreibung angegebene Bedeutung haben sowie mehrere Verfahren zu ihrer Herstellung.

Verwendet werden die neuen Phenoxyphenylsulfonylverbindungen als Herbizide und/oder als Pflanzenwuchsregulatoren.

EP 0 457 140 A2

Die Erfindung betrifft neue Phenoxyphenylsulfonylverbindungen, Verfahren zu ihrer Herstellung sowie ihre Verwendung als Herbizide und Pflanzenwuchsregulatoren.

Es ist bekannt, daß bestimmte Phenoxyphenylverbindungen, wie z. B. 3-(2,4-Dichlor-phenoxy)-6-nitrobenzoesäuremethylester (Bifenox) herbizid wirksam sind (vgl. US-P 3 652 645 und US-P 3 776 715). Die Wirkung dieser bekannten Verbindung ist jedoch nicht in allen Belangen zufriedenstellend.

Weiter sind auch einige Phenoxyphenylsulfonylverbindungen als potentielle Herbizide bekanntgeworden (vgl. DE-OS 2833021/US-P 4213775). Diese Verbindungen haben jedoch wegen unbefriedigender Wirkung keine praktische Bedeutung erlangt.

Es wurden nun neue Phenoxyphenylsulfonylverbindungen der allgemeinen Formel (I)

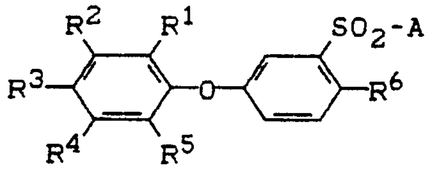

(I)

in welcher

$R^1$ für Wasserstoff, Halogen, Cyano oder Trifluormethyl steht,

$R^2$ für Wasserstoff oder Halogen steht,

$R^3$ für Halogen, Cyano, Trifluormethyl, Trifluormethoxy, Methylsulfonyl oder Trifluormethylsulfonyl steht,

$R^4$ für Wasserstoff oder Halogen steht,

$R^5$ für Wasserstoff oder Halogen steht,

$R^6$ für Wasserstoff, Halogen, Cyano, Nitro, Amino oder Trifluormethyl steht und

A für die Gruppierung

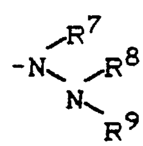

steht, wobei

$R^7$ für Wasserstoff oder Alkyl steht,

$R^8$ für Wasserstoff oder Alkyl steht und

$R^9$ für Wasserstoff, für jeweils gegebenenfalls substituiertes Alkyl, Cycloalkyl, Phenyl, Benzyl, Naphthyl, Pyridinyl, Pyrimidinyl oder Triazinyl steht, oder

$R^9$ für die Gruppierung $-CO-R^{10}$ steht, wobei

$R^{10}$ für Wasserstoff, Amino, Carbamoyl oder für jeweils gegebenenfalls substituiertes Alkyl, Cycloalkyl, Phenyl, Naphthyl, Pyridinyl, Furyl, Alkoxy, Phenoxy, Phenylamino oder Naphthyloxy steht, oder

$R^9$ für die Gruppierung $-SO_2-R^{11}$ steht, wobei

$R^{11}$ für jeweils gegebenenfalls substituiertes Alkyl, Phenyl, Naphthyl, Pyridinyl oder Thienyl steht, oder

$R^9$ zusammen mit $R^8$ für Alkandiyl steht;

oder

A für jeweils gegebenenfalls substituiertes Pyrrolidinyl, Piperidinyl, Perhydroazepinyl, Morpholinyl, Oxazolidinyl, Isoxazolidinyl, Perhydro-1,2-oxazinyl, Perhydro-1,3-oxazinyl oder Piperazinyl steht;

oder für den Fall, daß $R^3$ für Methylsulfonyl oder Trifluormethylsulfonyl steht

A auch für Amino, Alkylamino, Dialkylamino oder für gegebenenfalls substituiertes Azolyl steht,

gefunden.

Weiter wurde gefunden, daß man die neuen Phenoxyphenylsulfonylverbindungen der allgemeinen Formel (I) erhält, wenn man

(a) Phenoxybenzolsulfonsäurechloride der allgemeinen Formel (II)

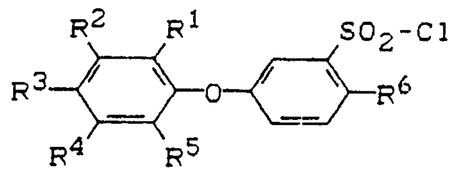

(II)

in welcher

$R^1$, $R^2$, $R^3$, $R^4$, $R^5$ und $R^6$ die oben angegebenen Bedeutungen haben,

mit Stickstoffverbindungen der allgemeinen Formel (III)

H-A (III)

in welcher

A die oben angegebene Bedeutung hat,

oder mit Alkalimetallsalzen von Verbindungen der Formel (III)

gegebenenfalls in Gegenwart eines Säureakzeptors und gegebenenfalls in Gegenwart eines Verdünnungsmittels umsetzt, oder wenn man

(b) für den Fall, daß in Formel (I) $R^6$ für Amino steht und $R^1$, $R^2$, $R^3$, $R^4$, $R^5$ und A die oben angegebenen Bedeutungen haben,

Verbindungen der allgemeinen Formel (I), in welcher $R^6$ für Nitro steht und $R^1$, $R^2$, $R^3$, $R^4$, $R^5$ und A die oben angegebenen Bedeutungen haben,

mit Wasserstoff in Gegenwart eines Hydrierungskatalysators und in Gegenwart eines Verdünnungsmittels umsetzt, oder wenn man

(c) für den Fall, daß in Formel (I) $R^6$ für Halogen oder Cyano steht und $R^1$, $R^2$, $R^3$, $R^4$, $R^5$ und A die oben angegebenen Bedeutungen haben,

Verbindungen der allgemeinen Formel (I), in welcher $R^6$ für Amino steht und $R^1$, $R^2$, $R^3$, $R^4$, $R^5$ und A die oben angegebenen Bedeutungen haben,

mit Natriumnitrit oder Kaliumnitrit und mit einem Hydrogenhalogenid (für $R^6$: Halogen) bzw. mit Schwefelsäure (für $R^6$: Cyano) in Gegenwart von Wasser und gegebenenfalls in Gegenwart eines organischen Lösungsmittels umsetzt und die hierbei erhaltenen Diazoniumsalzlösungen mit in Wasser gelösten Kupfer(I)-halogeniden bzw. mit Kupfer(I)-cyanid umsetzt.

Schließlich wurde gefunden, daß die neuen Phenoxyphenylsulfonylverbindungen der Formel (I) starke herbizide und pflanzenwuchsregulierende Wirkung zeigen.

Überraschenderweise sind die erfindungsgemäßen Phenoxyphenylsulfonylverbindungen der Formel (I) gegen Unkräuter wesentlich stärker wirksam als 3-(2,4-Dichlor-phenoxy)-6-nitro-benzoesäure-methylester, welches ein strukturell ähnlicher vorbekannter Wirkstoff gleicher Wirkungsrichtung ist.

Die Erfindung betrifft vorzugsweise Verbindungen der Formel (I), in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Cyano, Trifluormethyl, Trifluormethoxy, Methylsulfonyl oder Trifluormethylsulfonyl steht,

$R^4$ für Wasserstoff, Fluor oder Chlor steht,

$R^5$ für Wasserstoff, Fluor oder Chlor steht,

$R^6$ für Wasserstoff, Fluor, Chlor, Brom, Iod, Cyano, Nitro, Amino oder Trifluormethyl steht und

A für die Gruppierung

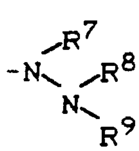

steht, wobei

$R^7$ für Wasserstoff oder Methyl steht,

$R^8$ für Wasserstoff oder $C_1$-$C_6$-Alkyl steht und

$R^9$ für Wasserstoff, für gegebenenfalls durch Hydroxy, Fluor und/oder Chlor substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, $C_1$-$C_4$-Alkyl und/oder $C_1$-$C_4$-Alkoxy substituiertes Phenyl, für Benzyl, für Naphthyl, für jeweils gegebenenfalls durch Fluor, Chlor, Brom, Methyl, Ethyl, Methoxy und/oder Ethoxy substituiertes Pyridinyl, Pyrimidinyl oder Triazinyl, oder

$R^9$ für die Gruppierung -CO-$R^{10}$ steht, wobei

$R^{10}$ für Wasserstoff, Amino, Carbamoyl, für gegebenenfalls durch Fluor, Chlor, Brom und/oder $C_1$-$C_4$-Alkoxy substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Hydroxy, Amino, Fluor, Chlor, Brom, Trifluormethyl, $C_1$-$C_4$-Alkyl und/oder $C_1$-$C_4$-Alkoxy substituiertes Phenyl, für gegebenenfalls durch Hydroxy substituiertes Naphthyl, für Pyridinyl oder Furyl, für $C_1$-$C_6$-Alkoxy, Phenoxy, Phenylamino oder Naphthyloxy steht, oder

$R^9$ für die Gruppierung -$SO_2$-$R^{11}$ steht, wobei

$R^{11}$ für gegebenenfalls durch Fluor und/oder Chlor substituiertes $C_1$-$C_6$-Alkyl, für jeweils gegebenenfalls durch Fluor, Chlor, Brom, Cyano, Nitro, Carboxy, Methyl, Trifluormethyl, Methoxy, Difluormethoxy, Trifluormethoxy, Dimethylaminosulfonyl, Methoxycarbonyl, Ethoxycarbonyl und/oder Dimethylaminocarbonyl substituiertes Phenyl oder Pyridinyl, für Naphthyl oder für gegebenenfalls durch Fluor, Chlor, Brom, Cyano, Nitro, Methyl, Methoxy und/oder Methoxycarbonyl substituiertes Thienyl steht, oder

$R^9$ zusammen mit $R^8$ für $C_4$-$C_6$-Alkandiyl steht;

oder

A für jeweils gegebenenfalls durch $C_1$-$C_4$-Alkyl, Formyl, $C_1$-$C_4$-Alkylcarbonyl, $C_1$-$C_4$-Alkoxycarbonyl, Phenyl und/ oder $C_1$-$C_4$-Alkoxy substituiertes Pyrrolidinyl, Piperidinyl, Perhydroazepinyl, Morpholinyl, Oxazolidinyl, Isoxazolidinyl, Perhydro-1,2-oxazinyl, Perhydro-1,3-oxazinyl oder Piperazinyl steht;

oder für den Fall, daß $R^3$ für Methylsulfonyl oder Trifluormethylsulfonyl steht

A auch für Amino, $C_1$-$C_6$-Alkylamino, Di-($C_1$-$C_4$-alkyl)-amino oder für gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Pyrazolyl, Imidazolyl oder Triazolyl steht.

Die Erfindung betrifft insbesondere Verbindungen der Formel (I), in welcher

$R^1$ für Wasserstoff, Fluor oder Chlor steht,

$R^2$ für Wasserstoff, Fluor oder Chlor steht,

$R^3$ für Trifluormethyl, Methylsulfonyl oder Trifluormethylsulfonyl steht,

$R^4$ für Wasserstoff, Fluor oder Chlor steht,

$R^5$ für Wasserstoff, Fluor oder Chlor steht,

$R^6$ für Wasserstoff, Chlor, Brom, Cyano oder Nitro steht und

A für die Gruppierung

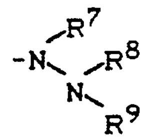

steht, wobei

$R^7$ für Wasserstoff steht,

$R^8$ für Wasserstoff oder Methyl steht und

$R^9$ für Wasserstoff, Methyl, Ethyl, Propyl, Isopropyl, Butyl, Isobutyl, sec-Butyl, tert-Butyl, Cyclopentyl, Cyclohexyl, Phenyl, Fluorphenyl, Chlorphenyl, Bromphenyl, Methylphenyl, Methoxyphenyl, für jeweils gegebenenfalls durch Chlor, Methyl und/oder Methoxy substituiertes Pyridinyl, Pyrimidinyl oder Triazinyl oder

$R^9$ für die Gruppierung -CO-$R^{10}$ steht, wobei

$R^{10}$ für Methyl, Ethyl, für gegebenenfalls durch Fluor, Chlor, Methyl und/oder Trifluormethyl substituiertes Phenyl, Pyridinyl, Furyl, Methoxy oder Ethoxy steht, oder

$R^9$ für die Gruppierung -$SO_2$-$R^{11}$ steht, wobei

$R^{11}$ für Methyl, Ethyl, Propyl, Butyl oder für gegebenenfalls einfach bis dreifach durch Methyl

substituiertes Phenyl steht, oder

$R^9$ zusammen mit $R^8$ für Tetramethylen, Pentamethylen oder Hexamethylen steht;

oder

A für jeweils gegebenenfalls einfach bis dreifach durch Methyl und/oder Ethyl substituiertes Pyrrolidinyl, Piperidinyl, Perhydroazepinyl, Morpholinyl, Oxazolidinyl, Isoxazolidinyl oder Piperazinyl, das gegebenenfalls auch durch Formyl, Acetyl, Methoxycarboxyl, Ethoxycarbonyl oder Phenyl substituiert ist, steht;

oder für den Fall, daß $R^3$ für Methylsulfonyl oder Trifluormethylsulfonyl steht

A auch für Amino, Methylamino, Ethylamino, Propylamino, Isopropylamino, Butylamino, Dimethylamino, Diethylamino oder für gegebenenfalls einfach bis dreifach, gleich oder verschieden durch Chlor, Brom, Methyl, Ethyl und/oder Trifluormethyl substituiertes Pyrazolyl oder Imidazolyl steht.

Beispiele für die neuen Phenoxyphenylsulfonylverbindungen der Formel (I) sind in der nachstehenden Tabelle 1 aufgeführt.

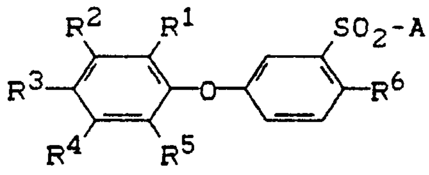 (I)

Tabelle 1

| $R^1$ | $R^2$ | $R^3$ | $R^4$ | $R^5$ | $R^6$ | A |
|---|---|---|---|---|---|---|
| Cl | H | $-SO_2CH_3$ | H | H | $NO_2$ | 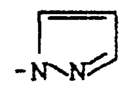 |
| Cl | H | $-SO_2CH_3$ | H | Cl | $NO_2$ | 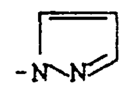 |
| Cl | H | $-SO_2CH_3$ | H | F | $NO_2$ | 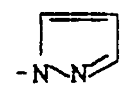 |
| Cl | H | $-SO_2CF_3$ | H | Cl | $NO_2$ | 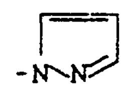 |
| Cl | H | $-SO_2CF_3$ | H | Cl | $NO_2$ | 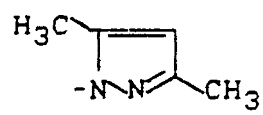 |
| Cl | H | $-SO_2CH_3$ | H | Cl | $NO_2$ | 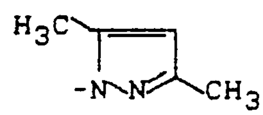 |
| Cl | H | $-SO_2CH_3$ | H | F | $NO_2$ | 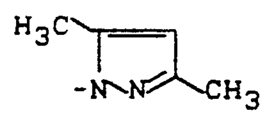 |
| Cl | H | $-SO_2CH_3$ | H | H | $NO_2$ | 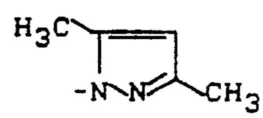 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $-SO_2CH_3$ | H | Cl | $NO_2$ | 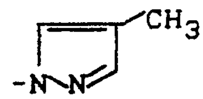 |
| Cl | H | $-SO_2CH_3$ | H | F | $NO_2$ | 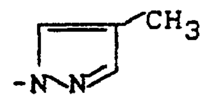 |
| Cl | H | $-SO_2CH_3$ | H | Cl | $NO_2$ | 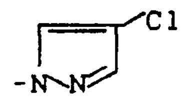 |
| Cl | H | $-SO_2CH_3$ | H | Cl | $NO_2$ | 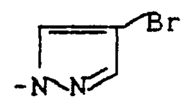 |
| Cl | H | $-SO_2CH_3$ | H | H | $NO_2$ | 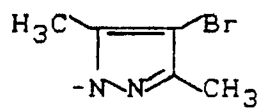 |
| Cl | H | $-SO_2CH_3$ | H | Cl | $NO_2$ | 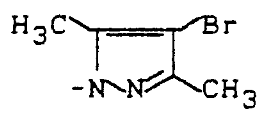 |
| Cl | H | $-SO_2CH_3$ | H | F | $NO_2$ | 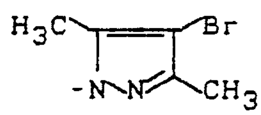 |
| Cl | H | $-SO_2CH_3$ | H | Cl | $NO_2$ | 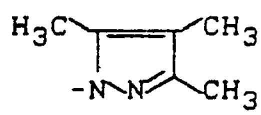 |
| Cl | H | $-SO_2CH_3$ | H | F | $NO_2$ | 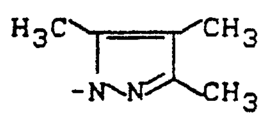 |
| Cl | H | $-SO_2CH_3$ | H | H | $NO_2$ | 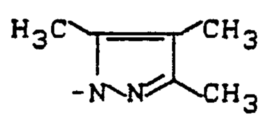 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $-SO_2CH_3$ | H | H | $NO_2$ | 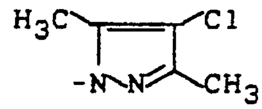 |
| Cl | H | $-SO_2CH_3$ | H | Cl | $NO_2$ | 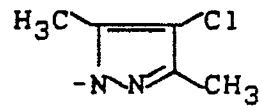 |
| Cl | H | $-SO_2CH_3$ | H | F | $NO_2$ | 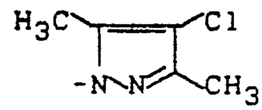 |
| Cl | H | $-SO_2CH_3$ | H | H | $NO_2$ | 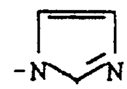 |
| Cl | H | $-SO_2CF_3$ | H | Cl | $NO_2$ | 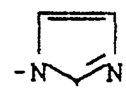 |
| Cl | H | $-SO_2CF_3$ | H | F | $NO_2$ | 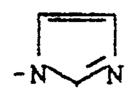 |
| Cl | H | $-SO_2CH_3$ | H | F | $NO_2$ | 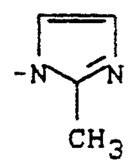 |
| Cl | H | $-SO_2CH_3$ | H | H | $NO_2$ | 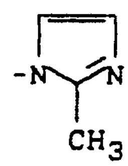 |
| Cl | H | $-SO_2CH_3$ | H | Cl | $NO_2$ | 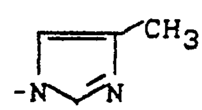 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $-SO_2CH_3$ | H | H | $NO_2$ | $NH_2$ |
| Cl | H | $-SO_2CH_3$ | H | H | $NO_2$ | $NHCH_3$ |
| Cl | H | $-SO_2CH_3$ | H | H | $NO_2$ | $NHC_2H_5$ |
| Cl | H | $-SO_2CH_3$ | H | H | $NO_2$ | $NHC_3H_7$ |
| Cl | H | $-SO_2CH_3$ | H | H | $NO_2$ | $NHCH(CH_3)_2$ |
| Cl | H | $-SO_2CH_3$ | H | H | $NO_2$ | $N(CH_3)_2$ |
| Cl | H | $-SO_2CH_3$ | H | H | $NO_2$ | $N(C_2H_5)_2$ |
| Cl | H | $CF_3$ | H | H | H | $-NHN(CH_3)_2$ |
| Cl | H | $CF_3$ | H | Cl | H | $-NHNHC(CH_3)_3$ |
| Cl | H | $CF_3$ | H | F | H | $-NHNHC_6H_5$ |
| Cl | H | $CF_3$ | H | Cl | H | $-NHNHCOCH_3$ |
| Cl | H | $CF_3$ | H | Cl | H | $-NHNHSO_2CH_3$ |
| Cl | H | $CF_3$ | H | H | H | 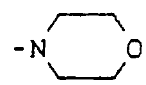 |
| Cl | H | $CF_3$ | H | Cl | H | 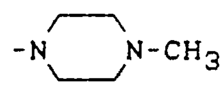 |
| Cl | H | $CF_3$ | H | Cl | H | 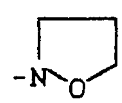 |
| Cl | H | $CF_3$ | H | H | Cl | -NHNH 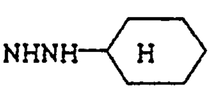 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $CF_3$ | H | Cl | Cl | 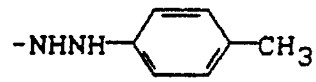 |
| Cl | H | $CF_3$ | H | F | Cl | $-NHNHCOC_6H_5$ |
| Cl | H | $CF_3$ | H | H | Cl | 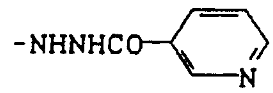 |
| Cl | H | $CF_3$ | H | H | Cl | $-NHNHSO_2C_6H_5$ |
| Cl | H | $CF_3$ | H | H | Br | $-NHNHSO_2C_4H_9$ |
| Cl | H | $CF_3$ | H | H | CN | $-NHNHC(CH_3)_3$ |
| Cl | H | $CF_3$ | H | H | Cl | 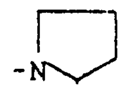 |
| Cl | H | $CF_3$ | H | H | Cl | 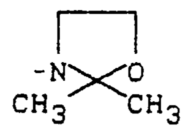 |
| Cl | H | $CF_3$ | H | Cl | Br | 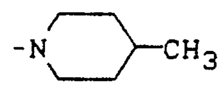 |
| Cl | H | $CF_3$ | H | H | CN | $-NHNHC_6H_5$ |
| Cl | H | $CF_3$ | H | Cl | CN | $-NHNHCOCH_3$ |
| Cl | H | $CF_3$ | H | F | CN | $-NHNHSO_2C_2H_5$ |
| Cl | H | $CF_3$ | H | H | CN | 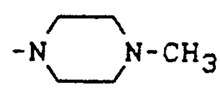 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $CF_3$ | H | F | CN | 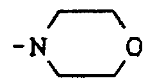 |
| Cl | H | $CF_3$ | H | H | CN | 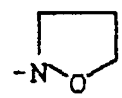 |
| Cl | H | $CF_3$ | H | Cl | CN | $-NHNHSO_2C_6H_5$ |
| Cl | H | $CF_3$ | H | F | CN | $-NHNHCOOC_2H_5$ |
| Cl | H | $CF_3$ | H | H | CN | 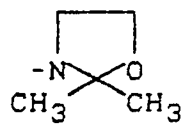 |
| Cl | H | $CF_3$ | H | H | $NO_2$ | $-NHN(CH_3)_2$ |
| Cl | H | $CF_3$ | H | Cl | $NO_2$ | $-NHNHC(CH_3)_3$ |
| Cl | H | $CF_3$ | H | F | $NO_2$ | $-NHNHC_6H_5$ |
| Cl | H | $CF_3$ | H | F | $NO_2$ | $-NHNHCOC_2H_5$ |
| Cl | H | $CF_3$ | H | H | $NO_2$ | $-NHNHSO_2CH_3$ |
| Cl | H | $CF_3$ | H | H | $NO_2$ | $-NHNHCOC_6H_5$ |
| Cl | H | $CF_3$ | H | Cl | $NO_2$ | $-NHNHSO_2C_6H_5$ |
| Cl | H | $CF_3$ | H | F | $NO_2$ | -NHNHCO 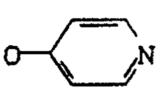 |
| Cl | H | $CF_3$ | H | H | $NO_2$ | -NHNHCO 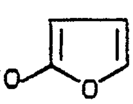 |
| Cl | H | $CF_3$ | H | F | $NO_2$ | 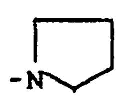 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $CF_3$ | H | F | $NO_2$ | 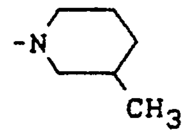 |
| Cl | H | $CF_3$ | H | H | $NO_2$ | 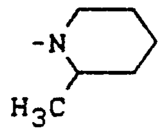 |
| Cl | H | $CF_3$ | H | Cl | $NO_2$ | 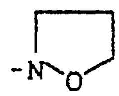 |
| Cl | H | $CF_3$ | H | H | $NO_2$ | 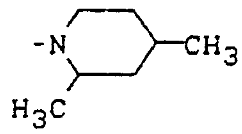 |
| Cl | H | $CF_3$ | H | H | $NO_2$ | 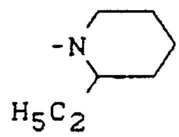 |
| Cl | H | $CF_3$ | H | F | $NO_2$ | 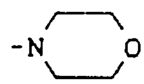 |
| Cl | H | $CF_3$ | H | F | $NO_2$ | 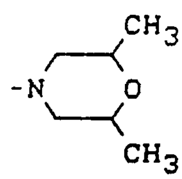 |
| Cl | H | $CF_3$ | H | F | $NO_2$ | 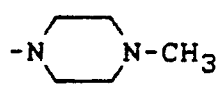 |
| Cl | H | $CF_3$ | H | H | $NO_2$ | 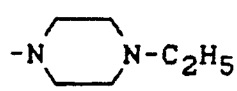 |

Tabelle 1 - Fortsetzung

| $R^1$ | $R^2$ | $R^3$ | $R^4$ | $R^5$ | $R^6$ | A |
|---|---|---|---|---|---|---|
| Cl | H | $CF_3$ | H | F | $NO_2$ | 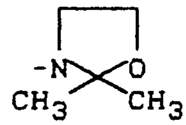 |
| Cl | H | $CF_3$ | H | Cl | $NO_2$ | 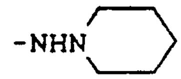 |
| Cl | H | $CF_3$ | H | F | $NO_2$ | 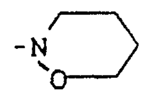 |

Verwendet man für das erfindungsgemäße Verfahren (a) beispielsweise 2-Cyano-5-(2,6-difluor-4-trifluormethylphenoxy)-benzolsulfonsäurechlorid und Acethydrazid als Ausgangsstoffe, so kann der Reaktionsablauf durch das folgende Formelschema wiedergegeben werden:

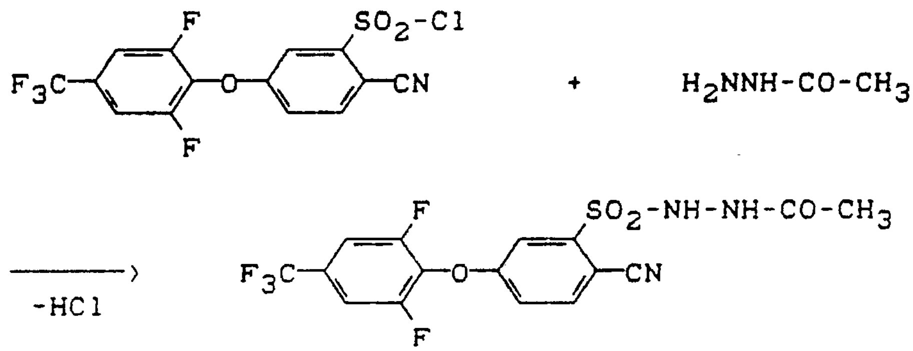


Verwendet man für das erfindungsgemäße Verfahren (b) beispielsweise N-(2-Nitro-5-(2-chlor-4-trifluormethylphenoxy)-phenyl-sulfonyl)-morpholin und Wasserstoff in Gegenwart eines Platin-Katalysators als Ausgangsstoffe, so kann der Reaktionsablauf durch das folgende Formelschema wiedergegeben werden:

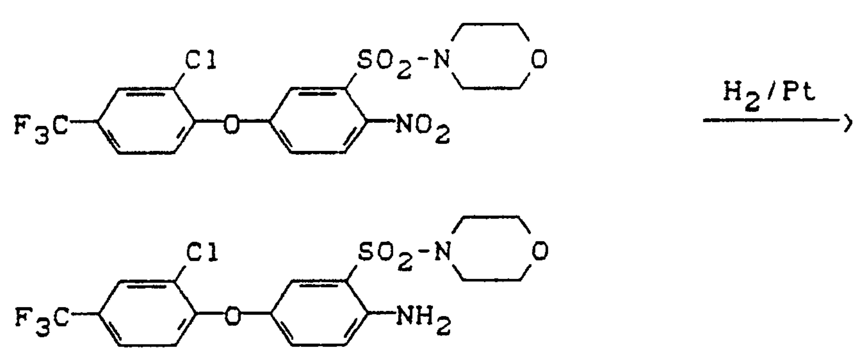


Verwendet man für das erfindungsgemäße Verfahren (c) beispielsweise N-(2-Amino-5-(2-chlor-6-fluor-4-trifluormethyl-phenoxy)-phenyl-sulfonyl)-pyrrolidin und Natriumnitrit/Hydrogenbromid sowie anschließend Kupfer(I)-bromid als Ausgangsstoffe, so kann der Reaktionsablauf durch das folgende Formelschema

wiedergegeben werden:

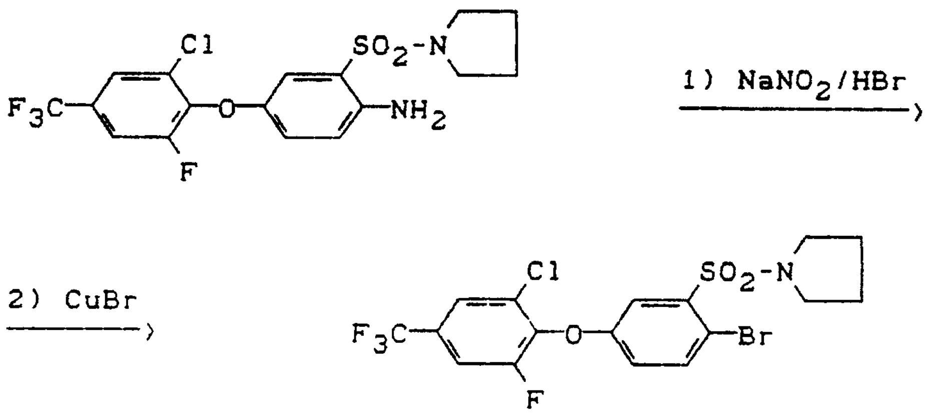


Die beim erfindungsgemäßen Verfahren (a) zur Herstellung von Verbindungen der Formel (I) als Ausgangsstoffe zu verwendenden Phenoxybenzolsulfonsäurechloride sind durch die Formel (II) allgemein definiert.

In Formel (II) haben $R^1$, $R^2$, $R^3$, $R^4$, $R^5$ und $R^6$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und $R^6$ angegeben wurden.

Als Beispiele für die Ausgangsstoffe der Formel (II) seien genannt:
3-(2-Chlor-4-trifluormethyl-phenoxy)-, 3-(2,6-Dichlor-4-trifluormethyl-phenoxy)-, 3-(2-Chlor-6-fluor-4-trifluormethyl-phenoxy)-, 3-(2,3,6-Trichlor-4-trifluormethyl)-phenoxy)- und 3-(2,6-Dichlor-3-fluor-4-trifluormethyl-phenoxy)-benzolsulfonsäurechlorid; 2-Chlor-, 2-Brom-, 2-Cyano- und 2-Nitro-5-(2-chlor-4-trifluormethyl-phenoxy)-, 2-Chlor-, 2-Brom-, 2-Cyano- und 2-Nitro-5-(2,6-dichlor-4-trifluormethyl-phenoxy-, 2-Chlor-, 2-Brom-, 2-Cyano- und 2-Nitro-5-(2-chlor-6-fluor-4-trifluormethyl-phenoxy)-, 2-Chlor-, 2-Brom-, 2-Cyano- und 2-Nitro-5-(2,3,6-trichlor-4-trifluormethyl-phenoxy)- und 2-Chlor-, 2-Brom-, 2-Cyano- und 2-Nitro-5-(2,6-dichlor-3-fluor-4-trifluormethyl-phenoxy)-benzolsulfonsäurechlorid.

Die Ausgangsstoffe der Formel (II) sind bekannt und/oder können nach an sich bekannten Verfahren hergestellt werden (vgl. US-P 4 213 775; DE-OS 3833549/LeA 26411).

Man erhält die Verbindungen der Formel (II) beispielsweise, wenn man
Aminodiphenylether der allgemeinen Formel (IV)

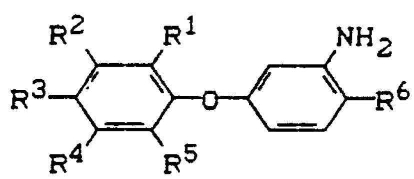
(IV)

in welcher

$R^1$, $R^2$, $R^3$, $R^4$ und $R^5$ die oben angegebenen Bedeutungen haben, und
$R^6$ mit Ausnahme von Amino die oben angegebene Bedeutung hat,

mit Natriumnitrit oder Kaliumnitrit in Gegenwart wäßriger Salzsäure und gegebenenfalls in Gegenwart eines organischen Lösungsmittels, wie z. B. Essigsäure, bei Temperaturen zwischen -10 °C und +20 °C umsetzt und die hierbei erhaltenen Diazoniumsalzlösungen mit Schwefeldioxid in Gegenwart eines Katalysators, wie z. B. Kupfer(I)- und/oder Kupfer(II)-chlorid, bei Temperaturen zwischen -10 °C und +40 °C umsetzt.

Die Aminodiphenylether der Formel (IV) sind bekannt und/oder können nach an sich bekannten Verfahren hergestellt werden (vgl. EP-A 29123; EP-A 313908/LeA 25624; DE-OS 3902288/LeA 26655).

Die beim erfindungsgemäßen Verfahren (a) weiter als Ausgangsstoffe zu verwendenden Stickstoffverbindungen sind durch die Formel (III) allgemein definiert. In Formel (III) hat A vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für A angegeben wurde. Bevorzugte Alkalimetallsalze der Verbindungen der Formel (III) sind deren Lithium-, Natrium- oder Kaliumsalze.

Als Beispiele für die Ausgangsstoffe der Formel (III) seien genannt:

Pyrazol, 4-Chlor-pyrazol, 4-Brom-pyrazol, 3-Methyl-, 4-Methyl-, 3,5-Dimethyl- und 3,4,5-Trimethyl-pyrazol, 4-Chlor-3,5-dimethyl-pyrazol, Imidazol, 2-Methyl- und 4-Methyl-imidazol, Hydrazin, Methylhydrazin, Ethylhydrazin, Propylhydrazin, Isopropylhydrazin, Butylhydrazin, Isobutylhydrazin, sec-Butylhydrazin, tert-Butylhydrazin, Dimethylhydrazin, Cyclopentylhydrazin, Cyclohexylhydrazin, Phenylhydrazin, 4-Fluor-phenylhydrazin, 4-Chlor-phenylhydrazin, 4-Brom-phenylhydrazin, 4-Methyl-phenylhydrazin, 4-Methoxy-phenylhydrazin, 2-Hydrazino-pyridin, 3-Hydrazino-pyridin, 4-Hydrazino-pyridin, 2-Hydrazino-pyrimidin, 4,5-Dichlor-2-hydrazino-pyrimidin, 2-Hydrazino-4,6-dimethyl-pyrimidin, 2-Hydrazino-4,6-dimethoxy-pyrimidin, 2-Hydrazino-4-methoxy-6-methyl-pyrimidin, 2-Hydrazino-4,6-dimethyl-s-triazin, 2-Hydrazino-4,6-dimethoxy-s-triazin, 2-Hydrazino-4-methoxy-6-methyl-s-triazin, Acetylhydrazin, Propionylhydrazin, Benzoylhydrazin, 4-Fluor-benzoylhydrazin, 4-Chlorbenzoyl-hydrazin, 4-Methyl-benzoyl-hydrazin, 2-Pyridinoyl-hydrazin, 3-Pyridinoyl-hydrazin, 4-Pyridinoyl-hydrazin, 2-Furoylhydrazin, Methoxycarbonylhydrazin, Ethoxycarbonylhydrazin, Methylsulfonylhydrazin, Phenylsulfonylhydrazin, 4-Methyl-phenylsulfonylhydrazin, N-Amino-piperidin, N-Amino-perhydroazepin, Pyrrolidin, 2-Methyl-pyrrolidin, Piperidin, 2-Methyl-, 3-Methyl-, 4-Methyl-, 2-Ethyl-, 2,4-Dimethyl- und 2,5-Dimethyl-piperidin, Perhydroazepin, Morpholin, 2,2-Dimethyl-oxazolidin, Isoxazolidin, Piperazin, N-Methyl- und N-Ethyl-piperazin.

Die Verbindungen der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden Verbindungen sind - mit der Maßgabe, daß jeweils $R^6$ für Nitro steht - durch die Formel (I) allgemein definiert.

In Formel (I) haben dann $R^1$, $R^2$, $R^3$, $R^4$, $R^5$ und A vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und A angegeben wurden.

Die Ausgangsstoffe der Formel (I) für Verfahren (b) sind erfindungsgemäße, neue Verbindungen; sie können nach den erfindungsgemäßen Verfahren (a) hergestellt werden.

Verfahren (b) wird unter Verwendung eines Hydrierungskatalysators durchgeführt. Als Beispiele hierfür seien Raney-Nickel, Platin und Palladium genannt. Vorzugsweise wird Raney-Nickel für Verfahren (b)

eingesetzt.

Verfahren (b) wird in Gegenwart eines Verdünnungsmittels durchgeführt. Vorzugsweise werden Alkohole, wie Methanol, Ethanol, Propanol oder Isopropanol, Ether, wie Glycoldimethylether, Diglycoldimethylether, Tetrahydrofuran oder Dioxan, oder Ester, wie Essigsäuremethylester oder Essigsäureethylester, als Lösungsmittel für Verfahren (b) eingesetzt.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (b) wird im allgemeinen bei Normaldruck oder erhöhtem Druck bis etwa 200 bar, vorzugsweise bis etwa 100 bar, durchgeführt.

Verfahren (b) kann unter den bei katalytischen Hydrierungen üblichen Bedingungen durchgeführt werden. In einer bevorzugten Ausführungsform von Verfahren (b) wird die Ausgangsverbindung der Formel (I) mit dem Verdünnungsmittel und dem Katalysator vermischt und dann so lange Wasserstoff zudosiert, bis kein Verbrauch von Wasserstoff mehr festzustellen ist. Nach dem Ende der Hydrierung filtriert man das Reaktionsgemisch und erhält nach Einengen des Filtrats das Rohprodukt als Rückstand, welcher auf übliche Weise, beispielsweise durch Säulenchromatographie, gereinigt werden kann.

Die beim erfindungsgemäßen Verfahren (c) zur Herstellung von Verbindungen der Formel (I) als Ausgangsstoffe zu verwendenden Verbindungen sind - mit der Maßgabe, daß jeweils $R^6$ für Amino steht - durch die Formel (I) allgemein definiert.

In Formel (I) haben dann $R^1$, $R^2$, $R^3$, $R^4$, $R^5$ und A vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und A angegeben wurden.

Die Ausgangsstoffe der Formel (I) für Verfahren (c) sind erfindungsgemäße, neue Verbindungen; sie können nach dem erfindungsgemäßen Verfahren (b) hergestellt werden.

Verfahren (c) wird unter Einsatz eines Hydrogenhalogenids bzw. von Schwefelsäure durchgeführt. Als Beispiele für die Hydrogenhalogenide seien Hydrogenfluorid, Hydrogenchlorid, Hydrogenbromid und Hydrogeniodid genannt.

Verfahren (c) wird gegebenenfalls unter Verwendung eines organischen Lösungsmittels durchgeführt. Geeignete organische Lösungsmittel sind beispielsweise Ether, wie Glycoldimethylether und Diglycoldimethylether, sowie Tetrahydrofuran und Dioxan, Ketone, wie Aceton und Methylethylketon, sowie Amide, wie Dimethylformamid.

Verfahren (c) wird unter Einsatz von Kupfer(I)-halogeniden bzw. von Kupfer(I)-cyanid durchgeführt. Als Beispiele für die Kupfer(I)-halogenide seien Kupfer(I)-chlorid, Kupfer(I)-bromid und Kupfer(I)-iodid genannt.

Die Reaktionstemperaturen können bei dem erfindungsgemäßen Verfahren (c) in einem größeren Bereich variiert werden. Im allgemeinen arbeitet man bei Temperaturen zwischen -20 °C und +80 °C, vorzugsweise bei Temperaturen zwischen 0 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man je Mol Ausgangsverbindung der Formel (I) im allgemeinen zwischen 0,8 und 2,5 Mol, vorzugsweise zwischen 1,1 und 1,5 Mol, Natriumnitrit oder Kaliumnitrit und zwischen 2 und 20 Mol, vorzugsweise zwischen 3 und 10 Mol, Hydrogenhalogenid bzw. Schwefelsäure, sowie zwischen 1 und 3 Mol, vorzugsweise zwischen 1,1 und 1,5 Mol, Kupfer(I)-halogenid bzw. Kupfer(I)-cyanid ein.

Zunächst wird auf übliche Weise eine Diazotierung durchgeführt: Man legt hierzu im allgemeinen die Ausgangsverbindung der Formel (I) in einer wäßrigen Lösung eines Hydrogenhalogenids bzw. von Schwefelsäure vor und gibt dazu unter Kühlen langsam eine wäßrige Lösung von Natriumnitrit oder Kaliumnitrit. Überschüssige salpetrige Säure wird gegebenenfalls nach der Diazotierung mit Harnstoff beseitigt und die Diazoniumsalzlösung wird unter Kühlen zu einer wäßrigen Lösung eines Kupfer(I)-halogenids bzw. von Kupfer(I)-cyanid gegeben. Dann wird, gegebenenfalls unter Erwärmen, bis zum Ende der Gasentwicklung gerührt und anschließend nach üblichen Methoden aufgearbeitet.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und insbesondere von dikotylen Unkräutern in monokotylen und dikotylen Kulturen sowohl im Vorauflauf- als auch im Nachauflauf-Verfahren. In gewissem Umfang zeigen sie auch insektizide Wirkung.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate

sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); 3,5-Dibrom-4-hydroxybenzonitril (BROMOXYNIL); N-(Butoxymethyl)-2-chlor-N-(2,6-diethylphenyl)-acetamid(BUTACHLOR); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxyl-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5-(4H)-on (ETHIOZIN); 2-{4-[(6-Chlor-2-benzoxazolyl)-oxylphenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxyl-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethylpyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethylphenyl)-4-methylamino-5-chlor-6-pyridazon (NORFLURAZON); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion

(SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

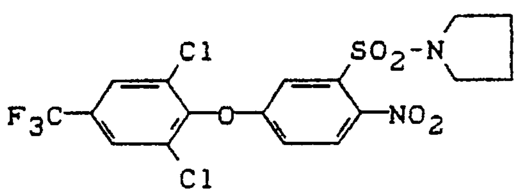

(Verfahren (a))

Eine Mischung aus 4,5 g (10 mMol) 2-Nitro-5-(2,6-dichlor-4-trifluormethyl-phenoxy)-benzolsulfonsäurechlorid, 1,6 g (22 mMol) Pyrrolidin und 60 ml Methylenchlorid wird 4 Stunden bei 20° C gerührt und anschließend eingeengt. Der Rückstand wird mit 20 ml 2N Salzsäure verrührt und das kristallin angefallene Produkt durch Absaugen isoliert.

Man erhält 4,8 g (99% der Theorie) N-(2-Nitro-5-(2,6-dichlor-4-trifluormethyl-phenoxy)-phenylsulfonyl)-pyrrolidin vom Schmelzpunkt 168° C.

Beispiel 2

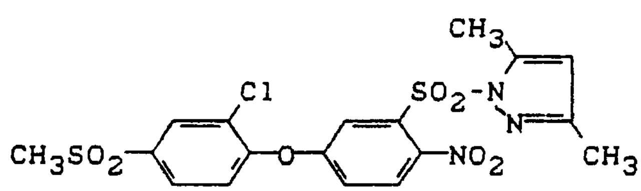

(Verfahren (a))

2,06 g (5 mMol) 2-Nitro-5-(2-chlor-4-methylsulfonylphenoxy)-benzolsulfonsäurechlorid werden in 50 ml

Methylenchlorid vorgelegt und unter Rühren nacheinander mit 0,5 g (5 mMol) Triethylamin und 0,45 g (5 mMol) 3,5-Dimethyl-pyrazol versetzt. Das Reaktionsgemisch wird 15 Stunden bei 20 °C gerührt, dann nach Verdünnen mit Methylenchlorid und Wasser durchgeschüttelt, die organische Phase abgetrennt, mit 2N-Salzsäure gewaschen, mit Natriumsulfat getrocknet und filtriert. Vom Filtrat wird das Lösungsmittel im Wasserstrahlvakuum sorgfältig abdestilliert.

Man erhält 1,1 g (45% der Theorie) 1-(2-Nitro-5-(2-chlor-4-methylsulfonyl-phenoxy)-phenylsulfonyl)-3,5-dimethyl-pyrazol als festen Rückstand vom Schmelzpunkt 125 °C.

Analog zu den Beispielen 1 oder 2 sowie entsprechend der allgemeinen Beschreibung der erfindungsgemäßen Herstellungsverfahren können beispielsweise auch die in der nachstehenden Tabelle 2 aufgeführten Verbindungen der Formel (I) erhalten werden.

**Tabelle 2:** Herstellungsbeispiele für die Verbindungen der Formel (I)

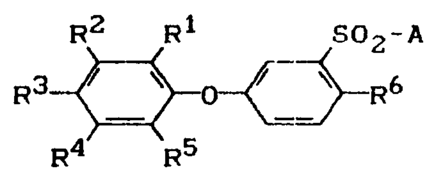

(I)

| Bsp.-Nr. | $R^1$ | $R^2$ | $R^3$ | $R^4$ | $R^5$ | $R^6$ | A | Physikalische Daten |
|---|---|---|---|---|---|---|---|---|
| 3 | Cl | H | $CF_3$ | H | Cl | $NO_2$ | 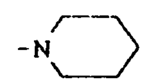 | Fp. 160° C |
| 4 | Cl | H | $CF_3$ | H | Cl | $NO_2$ | 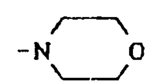 | Fp. 216° C |
| 5 | Cl | H | $CF_3$ | H | Cl | $NO_2$ | $-NHNHCOCH_3$ | Fp. 173° C |
| 6 | Cl | H | $CF_3$ | H | Cl | $NO_2$ | 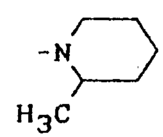 | Fp. 111° C |
| 7 | Cl | H | $CF_3$ | H | Cl | $NO_2$ | 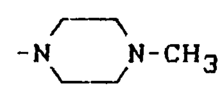 | Fp. 190° C |

Tabelle 2 - Fortsetzung

| Bsp.-Nr. | $R^1$ | $R^2$ | $R^3$ | $R^4$ | $R^5$ | $R^6$ | A | Physikalische Daten |
|---|---|---|---|---|---|---|---|---|
| 8 | Cl | H | $CF_3$ | H | Cl | $NO_2$ | 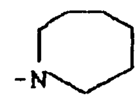 | Fp. 147° C |
| 9 | Cl | H | $CF_3$ | H | Cl | $NO_2$ | $-NHNHCOOC_2H_5$ | Fp. 149° C |
| 10 | Cl | H | $CF_3$ | H | Cl | $NO_2$ | $-NHNHSO_2CH_3$ | (amorph) |
| 11 | Cl | H | $CF_3$ | H | Cl | $NO_2$ | -NHNH- 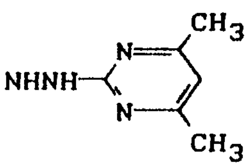 | Fp. 198° C |
| 12 | Cl | H | $CF_3$ | H | Cl | $NO_2$ | $-NHNHSO_2-$ 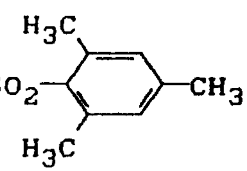 | Fp. 190° C |
| 13 | Cl | H | $-SO_2CH_3$ | H | H | $NO_2$ | $NH_2$ | Fp. 242° C |

Tabelle 2 - Fortsetzung

| Bsp.-Nr. | $R^1$ | $R^2$ | $R^3$ | $R^4$ | $R^5$ | $R^6$ | A | Physikalische Daten |
|---|---|---|---|---|---|---|---|---|
| 14 | Cl | H | $-SO_2CH_3$ | H | H | $NO_2$ | 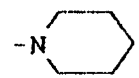 | Fp. 178° C |
| 15 | Cl | H | Cl | H | H | $NO_2$ | -NHNH- 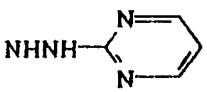 | Fp. 193° C |
| 16 | Cl | H | $CF_3$ | H | Cl | $NO_2$ | -NHNH-CO- 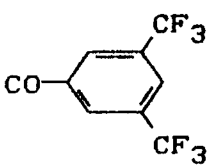 | Fp. 252° C |
| 17 | Cl | H | $CF_3$ | H | F | CN | $-NHNHCOCH_3$ | Fp. 192° C |
| 18 | Cl | H | $CF_3$ | H | F | CN | 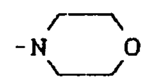 | Fp. 169° C |
| 19 | Cl | H | $CF_3$ | H | F | CN | -NHNH- 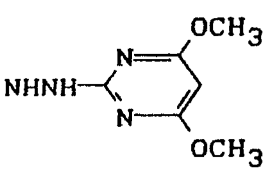 | Fp. 103° C |

**Tabelle 2** - Fortsetzung

| Bsp.-Nr. | $R^1$ | $R^2$ | $R^3$ | $R^4$ | $R^5$ | $R^6$ | A | Physikalische Daten |
|---|---|---|---|---|---|---|---|---|
| 20 | Cl | H | $CF_3$ | H | F | CN | 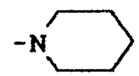 | Fp. 115° C |
| 21 | Cl | H | $CF_3$ | H | Cl | $NO_2$ | 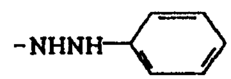 | Fp. 105° C |
| 22 | Cl | H | $CF_3$ | H | Cl | $NO_2$ | 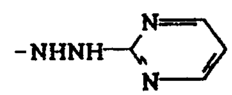 | Fp. 103° C |
| 23 | Cl | H | $CF_3$ | H | Cl | $NO_2$ | 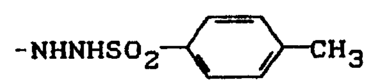 | Fp. 88° C |
| 24 | Cl | H | $CF_3$ | H | Cl | $NO_2$ | 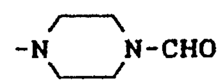 | Fp. 220° C |
| 25 | Cl | H | $CF_3$ | H | Cl | $NO_2$ | 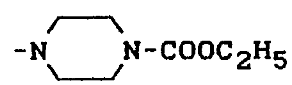 | Fp. 164° C |
| 26 | Cl | H | $CF_3$ | H | Cl | $NO_2$ | 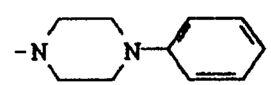 | Fp. 229° C |
| 27 | Cl | H | $CF_3$ | H | Cl | $NO_2$ | 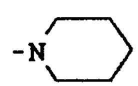 | Fp. 101° C |

Ausgangsstoffe der Formel (II):

Beispiel (II-1)

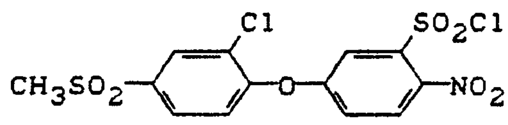

1. Stufe:

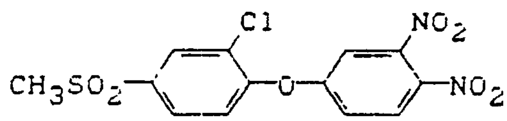

3,7 g (20 mMol) 4-Fluor-1,2-dinitro-benzol werden in 100 ml Dimethylsulfoxid auf 80 °C erhitzt und bei dieser Temperatur wird unter Rühren eine aus 4,1 g (20 mMol) 2-Chlor-4-methylsulfonyl-phenol, 1,9 g 45%iger Natronlauge (20 mMol NaOH) und 50 ml Dimethylsulfoxid hergestellte Lösung eindosiert. Das Gemisch wird 5 Stunden bei 80 °C gerührt, dann auf 600 ml Wasser gegossen und mit 2N Salzsäure angesäuert. Das kristallin angefallene Produkt wird durch Absaugen isoliert.

Man erhält 4,5 g (60% der Theorie) 1,2-Dinitro-4-(2-chlor-4-methylsulfonyl-phenoxy)-benzol vom Schmelzpunkt 109 °C.

2. Stufe:

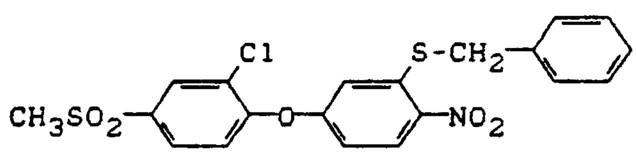

3,0 g (8 mMol) 1,2-Dinitro-4-(2-chlor-4-methylsulfonylphenoxy)-benzol werden in 50 ml Acetonitril vorgelegt und nacheinander mit 0,35 g Natriumhydroxid (gelöst in 2 ml Wasser) und 1,0 g (8,4 mMol) Benzylmercaptan versetzt. Das Gemisch wird eine Stunde bei 35 °C gerührt, dann auf 100 ml Wasser gegossen und mit 2N-Salzsäure angesäuert. Dann wird zweimal mit Essigsäureethylester extrahiert, die vereinigten organischen Phasen mit Natriumsulfat getrocknet und filtriert. Vom Filtrat wird das Lösungsmittel im Wasserstrahlvakuum sorfältig abdestilliert.

Man erhält 2,27 g (63% der Theorie) 1-Benzylthio-2-nitro-5-(2-chlor-4-methylsulfonyl-phenoxy)-benzol als festen Rückstand vom Schmelzpunkt 157 °C.

3. Stufe:

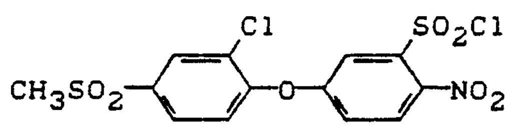

Eine Mischung aus 4,5 g (10 mMol) 1-Benzylthio-2-nitro-5-(2-chlor-4-methylsulfonyl-phenoxy)-benzol, 200 ml Essigsäure, 30 ml Wasser und 150 ml Methylenchlorid wird auf ca. 0 °C abgekühlt und in diese Mischung wird Chlor bis zur Bildung einer klaren, hellgelben Lösung eingeleitet. Die Mischung wird 15 Minuten gerührt und dann auf 1,5 Liter Wasser gegossen. Man extrahiert dann mit Methylenchlorid, wäscht die organische Phase mit Wasser, trocknet mit Natriumsulfat und filtriert. Vom Filtrat wird das Lösungsmittel im Wasserstrahlvakuum abdestilliert, der Rückstand mit Petrolether verrührt und das kristallin angefallene

Produkt durch Absaugen isoliert.

Man erhält 3,25 g (79% der Theorie) 2-Nitro-5-(2-chlor-4-methylsulfonyl-phenoxy)-benzolsulfonsäurechlorid vom Schmelzpunkt 108 °C.

Beispiel (II-2)

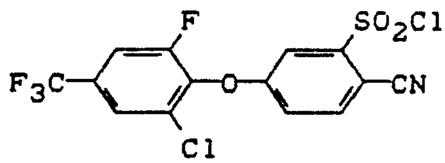

Eine Mischung aus 20,8 g (60 mMol) 2-Cyano-5-(2-chlor-6-fluor-4-trifluormethyl-phenoxy)-anilin, 100 ml Essigsäure und 120 ml konzentrierter Salzsäure wird auf 0 °C abgekühlt und tropfenweise mit einer Lösung von 4,6 g (66 mMol) Natriumnitrit in 8 ml Wasser versetzt. Das Reaktionsgemisch wird 90 Minuten bei 0 °C gerührt und anschließend zu einer gesättigten Lösung von Schwefeldioxid in 300 ml Essigsäure gegeben. Dann wird noch eine Lösung von 1,2 g Kupfer(II)-chlorid in 2 ml Wasser zudosiert und die Mischung wird 20 Stunden bei 10 °C bis 20 °C gerührt. Anschließend wird mit Eis verdünnt, mit Methylenchlorid geschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 10,8 g (43% der Theorie) 2-Cyano-5-(2-chlor-6-fluor-4-trifluormethyl-phenoxy)-benzolsulfonsäurechlorid als festen Rückstand vom Schmelzpunkt 130 °C.

Anwendungsbeispiele

In den folgenden Anwendungsbeispielen wird die nachstehend aufgeführte Verbindung als Vergleichssubstanz herangezogen:

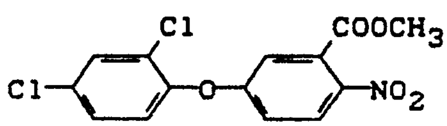

(A)

3-(2,4-Dichlor-phenoxy)-6-nitrobenzoesäure-methylester (bekannt aus US-PS 3 652 645 und US-PS 3 776 715).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine bis an 100 %ige Überlegenheit in der Wirksamkeit gegenüber der Vergleichssubstanz (A) zeigen

in diesem Test z.B. die Verbindungen gemäß den Herstellungsbeispielen: (1), (3), (4), (5), (6), (7), (9), (10), (11) und (17).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1), (5), (6), (7), (9), (10), (17) und (22) starke Wirkung gegen Unkräuter.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigen beispielsweise die Wirkstoffe gemäß den Herstellungsbeispielen (1), (3), (11) und (21) ein sehr starkes Austrocknen der Blätter und sehr starken Blattfall.

## Patentansprüche

1. Phenoxyphenylsulfonylverbindungen der allgemeinen Formel (I)

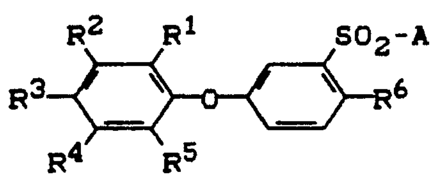

(I)

in welcher

- $R^1$ für Wasserstoff, Halogen, Cyano oder Trifluormethyl steht,
- $R^2$ für Wasserstoff oder Halogen steht,
- $R^3$ für Halogen, Cyano, Trifluormethyl, Trifluormethoxy, Methylsulfonyl oder Trifluormethylsulfonyl steht,
- $R^4$ für Wasserstoff oder Halogen steht,
- $R^5$ für Wasserstoff oder Halogen steht,
- $R^6$ für Wasserstoff, Halogen, Cyano, Nitro, Amino oder Trifluormethyl steht und

A für die Gruppierung

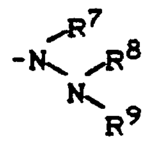

steht, wobei

$R^7$ für Wasserstoff oder Alkyl steht,

$R^8$ für Wasserstoff oder Alkyl steht und

$R^9$ für Wasserstoff, für jeweils gegebenenfalls substituiertes Alkyl, Cycloalkyl, Phenyl, Benzyl, Naphthyl, Pyridinyl, Pyrimidinyl oder Triazinyl steht, oder

$R^9$ für die Gruppierung $-CO-R^{10}$ steht, wobei

$R^{10}$ für Wasserstoff, Amino, Carbamoyl oder für jeweils gegebenenfalls substituiertes Alkyl, Cycloalkyl, Phenyl, Naphthyl, Pyridinyl, Furyl, Alkoxy, Phenoxy, Phenylamino oder Naphthyloxy steht, oder

$R^9$ für die Gruppierung $-SO_2-R^{11}$ steht, wobei

$R^{11}$ für jeweils gegebenenfalls substituiertes Alkyl, Phenyl, Naphthyl, Pyridinyl oder Thienyl steht, oder

$R^9$ zusammen mit $R^8$ für Alkandiyl steht;

oder

A für jeweils gegebenenfalls substituiertes Pyrrolidinyl, Piperidinyl, Perhydroazepinyl, Morpholinyl, Oxazolidinyl, Isoxazolidinyl, Perhydro-1,2-oxazinyl, Perhydro-1,3-oxazinyl oder Piperazinyl steht;

oder für den Fall, daS $R^3$ für Methylsulfonyl oder Trifluormethylsulfonyl steht

A auch für Amino, Alkylamino, Dialkylamino oder für gegebenenfalls substituiertes Azolyl steht.

2. Phenoxyphenylsulfonylverbindungen der Formel (I) gemäß Anspruch 1, in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Cyano, Trifluormethyl, Trifluormethoxy, Methylsulfonyl oder Trifluormethylsulfonyl steht,

$R^4$ für Wasserstoff, Fluor oder Chlor steht,

$R^5$ für Wasserstoff, Fluor oder Chlor steht,

$R^6$ für Wasserstoff, Fluor, Chlor, Brom, Iod, Cyano, Nitro, Amino oder Trifluormethyl steht und

A für die Gruppierung

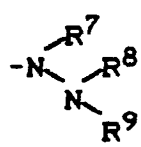

steht, wobei

$R^7$ für Wasserstoff oder Methyl steht,

$R^8$ für Wasserstoff oder $C_1$-$C_6$-Alkyl steht und

$R^9$ für Wasserstoff, für gegebenenfalls durch Hydroxy, Fluor und/oder Chlor substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, $C_1$-$C_4$-Alkyl und/oder $C_1$-$C_4$-Alkoxy substituiertes Phenyl, für Benzyl, für Naphthyl, für jeweils gegebenenfalls durch Fluor, Chlor, Brom, Methyl, Ethyl, Methoxy und/oder Ethoxy substituiertes Pyridinyl, Pyrimidinyl oder Triazinyl, oder

$R^9$ für die Gruppierung $-CO-R^{10}$ steht, wobei

$R^{10}$ für Wasserstoff, Amino, Carbamoyl, für gegebenenfalls durch Fluor, Chlor, Brom und/oder $C_1$-$C_4$-Alkoxy substituiertes $C_1$-$C_6$-Alkyl, für gegebenenfalls durch Fluor, Chlor, Brom

und/oder $C_1$-$C_4$-Alkyl substituiertes $C_3$-$C_6$-Cycloalkyl, für gegebenenfalls durch Hydroxy, Amino, Fluor, Chlor, Brom, Trifluormethyl, $C_1$-$C_4$-Alkyl und/ oder $C_1$-$C_4$-Alkoxy substituiertes Phenyl, für gegebenenfalls durch Hydroxy substituiertes Naphthyl, für Pyridinyl oder Furyl, für $C_1$-$C_6$-Alkoxy, Phenoxy, Phenylamino oder Naphthyloxy steht, oder

$R^9$ für die Gruppierung -$SO_2$-$R^{11}$ steht, wobei

$R^{11}$ für gegebenenfalls durch Fluor und/oder Chlor substituiertes $C_1$-$C_6$-Alkyl, für jeweils gegebenenfalls durch Fluor, Chlor, Brom, Cyano, Nitro, Carboxy, Methyl, Trifluormethyl, Methoxy, Difluormethoxy, Trifluormethoxy, Dimethylaminosulfonyl, Methoxycarbonyl, Ethoxycarbonyl und/oder Dimethylaminocarbonyl substituiertes Phenyl oder Pyridinyl, für Naphthyl oder für gegebenenfalls durch Fluor, Chlor, Brom, Cyano, Nitro, Methyl, Methoxy und/oder Methoxycarbonyl substituiertes Thienyl steht, oder

$R^9$ zusammen mit $R^8$ für $C_4$-$C_6$-Alkandiyl steht;

oder

A für jeweils gegebenenfalls durch $C_1$-$C_4$-Alkyl, Formyl, $C_1$-$C_4$-Alkylcarbonyl, $C_1$-$C_4$-Alkoxycarbonyl, Phenyl und/ oder $C_1$-$C_4$-Alkoxy substituiertes Pyrrolidinyl, Piperidinyl, Perhydroazepinyl, Morpholinyl, Oxazolidinyl, Isoxazolidinyl, Perhydro-1,2-oxazinyl, Perhydro-1,3-oxazinyl oder Piperazinyl steht;

oder für den Fall, daß $R^3$ für Methylsulfonyl oder Trifluormethylsulfonyl steht

A auch für Amino, $C_1$-$C_6$-Alkylamino, Di-($C_1$-$C_4$-alkyl)-amino oder für gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Pyrazolyl, Imidazolyl oder Triazolyl steht.

3. Phenoxyphenylsulfonylverbindungen der Formel (I) gemäß Anspruch 1, in welcher

$R^1$ für Wasserstoff, Fluor oder Chlor steht,

$R^2$ für Wasserstoff, Fluor oder Chlor steht,

$R^3$ für Trifluormethyl, Methylsulfonyl oder Trifluormethylsulfonyl steht,

$R^4$ für Wasserstoff, Fluor oder Chlor steht,

$R^5$ für Wasserstoff, Fluor oder Chlor steht,

$R^6$ für Wasserstoff, Chlor, Brom, Cyano oder Nitro steht und

A für die Gruppierung

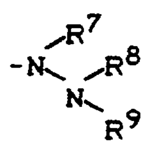

steht, wobei

$R^7$ für Wasserstoff steht,

$R^8$ für Wasserstoff oder Methyl steht und

$R^9$ für Wasserstoff, Methyl, Ethyl, Propyl, Isopropyl, Butyl, Isobutyl, sec-Butyl, tert-Butyl, Cyclopentyl, Cyclohexyl, Phenyl, Fluorphenyl, Chlorphenyl, Bromphenyl, Methylphenyl, Methoxyphenyl, für jeweils gegebenenfalls durch Chlor, Methyl und/oder Methoxy substituiertes Pyridinyl, Pyrimidinyl oder Triazinyl oder

$R^9$ für die Gruppierung -CO-$R^{10}$ steht, wobei

$R^{10}$ für Methyl, Ethyl, für gegebenenfalls durch Fluor, Chlor, Methyl und/oder Trifluormethyl substituiertes Phenyl, Pyridinyl, Furyl, Methoxy oder Ethoxy steht, oder

$R^9$ für die Gruppierung -$SO_2$-$R^{11}$ steht, wobei

$R^{11}$ für Methyl, Ethyl, Propyl, Butyl oder für gegebenenfalls einfach bis dreifach durch Methyl substituiertes Phenyl steht, oder

$R^9$ zusammen mit $R^8$ für Tetramethylen, Pentamethylen oder Hexamethylen steht;

oder

A für jeweils gegebenenfalls einfach bis dreifach durch Methyl und/oder Ethyl substituiertes Pyrrolidinyl, Piperidinyl, Perhydroazepinyl, Morpholinyl, Oxazolidinyl, Isoxazolidinyl oder Piperazinyl, das gegebenenfalls auch durch Formyl, Acetyl, Methoxycarbonyl, Ethoxycarbonyl oder Phenyl substituiert ist, steht;

oder für den Fall, daß $R^3$ für Methylsulfonyl oder Trifluormethylsulfonyl steht

A auch für Amino, Methylamino, Ethylamino, Propylamino, Isopropylamino, Butylamino, Dimethylamino, Diethylamino oder für gegebenenfalls einfach bis dreifach, gleich oder verschieden durch Chlor, Brom, Methyl, Ethyl und/oder Trifluormethyl substituiertes Pyrazolyl oder Imidazolyl steht.

**4.** Verfahren zur Herstellung von Phenoxyphenylsulfonylverbindungen der allgemeinen Formel (I)

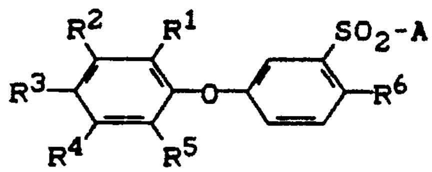

(I)

in welcher

$R^1$ für Wasserstoff, Halogen, Cyano oder Trifluormethyl steht,

$R^2$ für Wasserstoff oder Halogen steht,

$R^3$ für Halogen, Cyano, Trifluormethyl, Trifluormethoxy, Methylsulfonyl oder Trifluormethylsulfonyl steht,

$R^4$ für Wasserstoff oder Halogen steht,

$R^5$ für Wasserstoff oder Halogen steht,

$R^6$ für Wasserstoff, Halogen, Cyano, Nitro, Amino oder Trifluormethyl steht und

A für die Gruppierung

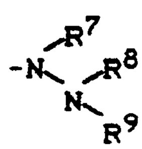

steht, wobei

$R^7$ für Wasserstoff oder Alkyl steht,

$R^8$ für Wasserstoff oder Alkyl steht und

$R^9$ für Wasserstoff, für jeweils gegebenenfalls substituiertes Alkyl, Cycloalkyl, Phenyl, Benzyl, Naphthyl, Pyridinyl, Pyrimidinyl oder Triazinyl steht, oder

$R^9$ für die Gruppierung -CO-$R^{10}$ steht, wobei

$R^{10}$ für Wasserstoff, Amino, Carbamoyl oder für jeweils gegebenenfalls substituiertes Alkyl, Cycloalkyl, Phenyl, Naphthyl, Pyridinyl, Furyl, Alkoxy, Phenoxy, Phenylamino oder Naphthyloxy steht, oder

$R^9$ für die Gruppierung -$SO_2$-$R^{11}$ steht, wobei

$R^{11}$ für jeweils gegebenenfalls substituiertes Alkyl, Phenyl, Naphthyl, Pyridinyl oder Thienyl steht, oder

$R^9$ zusammen mit $R^8$ für Alkandiyl steht;

oder

A für jeweils gegebenenfalls substituiertes Pyrrolidinyl, Piperidinyl, Perhydroazepinyl, Morpholinyl, Oxazolidinyl, Isoxazolidinyl, Perhydro-1,2-oxazinyl, Perhydro-1,3-oxazinyl oder Piperazinyl steht;

oder für den Fall, daß $R^3$ für Methylsulfonyl oder Trifluormethylsulfonyl steht

A auch für Amino, Alkylamino, Dialkylamino oder für gegebenenfalls substituiertes Azolyl steht,

dadurch gekennzeichnet, daß man

(a) Phenoxybenzolsulfonsäurechloride der allgemeinen Formel (II)

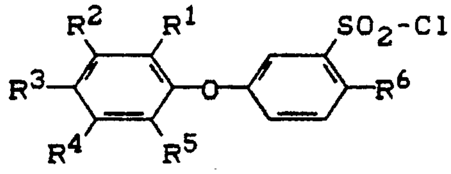

(II)

in welcher
$R^1$, $R^2$, $R^3$, $R^4$, $R^5$ und $R^6$ die oben angegebenen Bedeutungen haben,
mit Stickstoffverbindungen der allgemeinen Formel (III)

H-A (III)

in welcher
A die oben angegebene Bedeutung hat,
oder mit Alkalimetallsalzen von Verbindungen der Formel (III)
gegebenenfalls in Gegenwart eines Säureakzeptors und gegebenenfalls in Gegenwart eines Verdünnungsmittels umsetzt, oder
(b) für den Fall, daß in Formel (I) $R^6$ für Amino steht und $R^1$, $R^2$, $R^3$, $R^4$, $R^5$ und A die oben angegebenen Bedeutungen haben,
Verbindungen der allgemeinen Formel (I), in welcher $R^6$ für Nitro steht und $R^1$, $R^2$, $R^3$, $R^4$, $R^5$ und A die oben angegebenen Bedeutungen haben,
mit Wasserstoff in Gegenwart eines Hydrierungskatalysators und in Gegenwart eines Verdünnungsmittels umsetzt, oder
(c) für den Fall, daS in Formel (I) $R^6$ für Halogen oder Cyano steht und $R^1$, $R^2$, $R^3$, $R^4$, $R^5$ und A die oben angegebenen Bedeutungen haben,
Verbindungen der allgemeinen Formel (I), in welcher $R^6$ für Amino steht und $R^1$, $R^2$, $R^3$, $R^4$, $R^5$ und A die oben angegebenen Bedeutungen haben,
mit Natriumnitrit oder Kaliumnitrit und mit einem Hydrogenhalogenid (für $R^6$: Halogen) bzw. mit Schwefelsäure (für $R^6$: Cyano) in Gegenwart von Wasser und gegebenenfalls in Gegenwart eines organischen Lösungsmittels umsetzt und die hierbei erhaltenen Diazoniumsalzlösungen mit in Wasser gelösten Kupfer(I)-halogeniden bzw. Kupfer(I)-cyanid versetzt.

5. Herbizide und pflanzenwuchsregulierende Mittel, gekennzeichnet durch einen Gehalt an mindestens einer Phenoxyphenylsulfonylverbindung der Formel (I) nach den Ansprüchen 1 bis 4.

6. Verwendung von Phenoxyphenylsulfonylverbindungen der Formel (I) nach den Ansprüchen 1 bis 4 zur Bekämpfung von Unkräutern und/oder als Pflanzenwuchsregulator.

7. Verfahren zur Bekämpfung von Unkräutern und zur Regulierung des Pflanzenwachstums, dadurch gekennzeichnet, daß man Phenoxyphenylsulfonylverbindungen der Formel (I) nach den Ansprüchen 1 bis 4 auf die Unkräuter bzw. Pflanzen und/oder ihren Lebensraum einwirken läßt.

8. Verfahren zur Herstellung von herbiziden und/oder pflanzenwuchsregulierenden Mitteln, dadurch gekennzeichnet, daß man Phenoxyphenylsulfonylverbindungen der Formel (I) nach den Ansprüchen 1 bis 4 mit Streckmitteln und/oder oberflächenaktiven Mitteln vermischt.